# EUROPEAN PATENT SPECIFICATION

(11) **EP 4 223 237 B1**
(45) Date of publication and mention of the grant of the patent: **13.08.2025**
(21) Application number: 23153833.1
(22) Date of filing: 29.01.2023
(51) Int. Cl.: A61B 17/16, A61F 2/46

(54) **ACETABULAR CUP REMOVER ASSEMBLY**
HÜFTGELENKPFANNENENTFERNERANORDNUNG
ENSEMBLE DE RETRAIT DE CUPULE ACÉTABULAIRE

(30) Priority: 07.02.2022 US 202263267618 P; 11.01.2023 US 202318153127
(43) Date of publication of application: 09.08.2023
(62) Divisional of application: 23199853.5
(73) Proprietor: MicroPort Orthopedics Holdings Inc., Arlington, TN 38002 (US)
(72) Inventor: BARFIELD, Charles, Memphis, 38103 (US)
(74) Representative: Novagraaf Group

(56) References cited:
- WO-A1-2012/010366
- WO-A1-2013/046172
- WO-A1-2017/180602
- KR-B1- 101 402 893
- US-A1- 2008 009 874

## Description

### BACKGROUND OF THE INVENTION

### 1. Technical Field

The present disclosure relates to instruments for orthopedic surgery, and more particularly to instruments for the removal of acetabular hip cup implants in total hip arthroplasties.

### 2. Related Art

In a total hip arthroplasty ("THA"), deteriorated cartilage in the acetabulum is replaced with an artificial hip cup. The artificial hip cup interacts with a ball head of an artificial hip stem to provide an artificial hip joint. In some hip implant designs, the cup consists of a unibody cup that is secured in the acetabulum, such as by bone ingrowth, bone cement, or screws. In other hip implant designs, the cup comprises an outer shell that is secured in the acetabulum in the foregoing manner and a separate cup liner that is inserted in the shell between the outer shell and the ball head of the artificial hip stem.

The initial or "primary" hip implant typically lasts in a patient for about ten to twenty years. When the primary hip implant wears out, it is often useful to "revise" the primary hip implant by replacing it with new components. Revision typically includes removal and replacement of the primary acetabular cup. Additionally, when a revision cup wears out, it is typically desirable to remove and replace it.

Examples of prior cup removers that use a cutting blade to loosen the hip cup from the acetabulum include the cup removers disclosed in PCT App. No. WO2015/155657 (to Giardiello, et. al) and US Patent 6,565,575 (to Lewis). However, these designs are intended for use in conventional THAs. A THA is typically performed through a relatively large incision (*e.g*., between about 20 centimeters ("cm") to about 30.5 cm (8 to 12 inches)) to provide sufficient access to the j oint. The large incision also permits the introduction and manipulation of instruments within the joint. Large incisions may increase operating time and cause patients to lose substantial amounts of blood, inflict significant trauma to surrounding tissues (*e.g*., the nerves and muscles), increase the risk of infection, and require longer recovery periods.

Furthermore, the Giardiello and Lewis devices have modular cutting blades of different lengths and curvatures. A short thick blade may be used to start an opening between the hip cup and the acetabulum, while a longer increasingly curved blade may be used to expand the opening and remove the hip cup. These devices require that the cup remover be partially disassembled prior to replacing the modular cutting blades. This practice increases procedure duration and therefore necessarily increases the surgery's ancillary risks, including the infection risk and the possibility of complications arising from increased time under anesthesia.

In recent years, efforts have been made to develop "minimally invasive" procedures that reduce the incision length required for THA, with the aim of reducing blood loss, trauma, risk of infection, and recovery time. See U.S. Patent No. 6,905,502 (to Brad Penenberg, M.D.) and its family members (*e.g*., U.S. Patent Nos. 6,997,928; 7,833,229; and 6,997,928). These patents describe the earliest efforts to use a small portal incision in conjunction with a larger main incision in order to prepare the acetabulum for receipt of a hip implant using a posterior approach. Refinements of these instruments and methods are described in U.S. Patent 7,651,501 (to Brad Penenberg, M.D.) and its progeny (*e.g.*, U.S. Patent Nos. 8,439,928; 9,180,023; and 9,539,113). In these methods, a specially configured guide is placed into the main incision. A portion of the guide is placed in the acetabulum to provide a reference point. An outrigger structure extends from the guide outside of the main incision. An outer portion of the outrigger includes a guide for guiding instruments such as trocars and cannulas into alignment with the acetabulum. The guide is used to form a small posterior portal incision in alignment with the acetabulum, as well as to hold a cannula and to guide instruments during preparation of the acetabulum. By using a small posterior portal incision in conjunction with a main incision, the length of the main incision can be reduced to about the size of the acetabular cup, such as about 5 cm to about 7.6 cm (2 to 3 inches). Finally, US 2008/0009874 A1 discloses an apparatus for reaming an acetabulum through a single large incision.

However, when it comes to revision procedures, cup removal instruments have remained wedded to the single incision concept. There is a need for cup removers that can be used in minimally invasive two-incision techniques. Thus, there is a need for the techniques described herein below, which enable a reduced incision, good visualization, and other benefits that have not been obtained in conventional revision procedures.

### SUMMARY OF THE INVENTION

The problems associated with lengthy procedure duration and a large incision in a revision hip arthroplasty, including but not necessarily limited to an increased risk of patient infection, significant blood loss, trauma to the surrounding tissue, increased healing times, and complications arising from prolonged time under anesthesia can be mitigated by the assemblies or methods, or a combination of assemblies and methods in accordance with this disclosure. One such exemplary medical device assembly can comprise: an orientation bearing having an inner surface defining a hole, a positioning member engaged to the orientation bearing, the positioning member having a longitudinal body extending between a leading end and a trailing end, a cutting assembly having a mating portion configured to be rotatably disposed within the hole of the orientation bearing, wherein the cutting assembly comprises a cutting blade, and, a drive member having a drive member body extending between a drive member leading end and a drive member trailing end, wherein the drive member trailing end is configured to engage the cutting assembly to rotatably drive the cutting assembly. The present invention is defined in the appended claims.

It is contemplated that a medical device assembly in accordance with the present disclosure may provide an acetabular cup remover instrument assembly for use in hip arthroplasty revision procedures.

It is contemplated that a medical device assembly in accordance with the present disclosure may provide a cup remover assembly configured for use in minimally invasive hip procedures carried out through a primary and a portal incision.

It is further contemplated that a medical device assembly in accordance with the present disclosure may provide an acetabular cup remover assembly configured for use with a cannula in hip arthroplasty procedures.

Methods of using the instrument assembly and presenting the parts of the exemplary assemblies in kits are also described.

The foregoing and other features, aspects and advantages of the invention will become more apparent from the following detailed description of the invention when considered in conjunction with the accompanying drawings.

### BRIEF DESCRIPTION OF THE DRAWINGS

**FIG. 1** is a front-side perspective view of one exemplary embodiment of an acetabular cup remover assembly in the assembled configuration featuring a single fixed cutting blade.
**FIG. 2A** is a front-side perspective view of one exemplary embodiment of an acetabular cup remover assembly in the assembled configuration featuring an extendable cutting blade.
**FIG. 2B** is an exploded perspective view of the exemplary acetabular cup remover assembly of **FIG. 2A** in the disassembled configuration.
**FIG. 2C** is a detailed perspective view of the exemplary cutting assembly and exemplary cutting assembly support of **FIGS. 2A** and **2B** and the acetabular shell implant.
**FIG. 3** is a front-side perspective view of an exemplary embodiment of an acetabular cup remover assembly in the assembled configuration featuring an extendable cutting blade, with the extension of the cutting blade timed to the rotation of a handle.
**FIG. 4A** is a side perspective view of an exemplary embodiment of a cup remover assembly in a partially assembled configuration featuring a hemispherical cutting blade.
**FIG. 4B** is a side perspective view of the hemispherical cutting blade of **FIG. 4A** showing the hemispherical cutting blade aligned over the top of an acetabular shell implant (*i.e.*, a hip cup).
**FIG. 4C** is a perspective side view of the hemispherical cutting blade of **FIG. 4A** showing the hemispherical cutting blade aligned to cut around the side of the acetabular shell implant.
**FIG. 4D** is a close up perspective view of the hemispherical cutting blade of **FIG. 4A****,** which further details serrated teeth extending from a circumference of the hemispherical cutting blade.
**FIG. 4E** is a close up side cross sectional view of the hemispherical cutting blade of **FIG. 4A** further detailing a cross section of a serrated tooth to further illustrate the outer surface angle.
**FIG. 5** is a perspective side view of another exemplary embodiment of a cup remover assembly in the assembled configuration featuring a hemispherical cutting blade, an orientation bearing integrally engaged to the leading end of a positioning member, and a cutting assembly support rotatably engaged to the drive member via a universal joint.
**FIG. 6** is a side perspective view of an exemplary embodiment of a cup remover assembly in the assembled configuration in which the positioning member further comprises an articulating clamp comprising a first arm and a second arm configured to indirectly engage the orientation bearing.
**FIG. 7** is a side perspective view of an exemplary embodiment of a cup remover assembly in the assembled configuration similar to the embodiment of **FIG. 6** but having a more ergonomic handle.

### DETAILED DESCRIPTION OF THE PREFERRED EMBODIMENTS

In the following detailed description of the preferred embodiments, reference is made to the accompanying drawings which form a part hereof, and in which are shown by way of illustration, specific embodiments in which the invention may be practiced. It is to be understood that other embodiments may be utilized and structural changes may be made without departing from the scope of the appended claims.

The following detailed description of the preferred embodiments is presented only for illustrative and descriptive purposes and is not intended to be exhaustive or to limit the scope of the invention. The embodiments were selected and described to best explain the principles of the invention and its practical application. One of ordinary skill in the art will recognize that many variations can be made to the invention disclosed in this specification without departing from the scope of the claims.

Similar reference characters indicate corresponding parts throughout the several views unless otherwise stated. Although the drawings represent embodiments of various features and components according to the present disclosure, the drawings are not necessarily to scale and certain features may be exaggerated to better illustrate embodiments of the present disclosure, and such exemplifications are not to be construed as limiting the scope of the present disclosure.

Except as otherwise expressly stated herein, the following rules of interpretation apply to this specification: (a) all words used herein shall be construed to be of such gender or number (singular or plural) as such circumstances require; (b) the singular terms "a," "an," and "the," as used in the specification and the appended claims include plural references unless the context clearly dictates otherwise; (c) the antecedent term "about" applied to a recited range or value denotes an approximation with the deviation in the range or values known or expected in the art from the measurements; (d) the words, "herein," "hereby," "hereto," "hereinbefore," and "hereinafter," and words of similar import, refer to this specification in its entirety and not to any particular paragraph, claim, or other subdivision, unless otherwise specified; (e) descriptive headings are for convenience only and shall not control or affect the meaning of construction of part of the specification; and (f) "or" and "any" are not exclusive and "include" and "including" are not limiting. Further, the terms, "comprising," "having," "including," and "containing" are to be construed as open-ended terms (*i.e.*, meaning "including but not limited to").

References in the specification to "one embodiment," "an embodiment," "an exemplary embodiment," *etc.*, indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments, whether explicitly described.

To the extent necessary to provide descriptive support, the subject matter and/or text of the appended claims are incorporated herein by reference in their entirety.

Recitation of ranges of values herein are merely intended to serve as a shorthand method of referring individually to each separate value falling within the range of any sub-ranges there between, unless otherwise clearly indicated herein. Each separate value within a recited range is incorporated into the specification or claims as if each separate value were individually recited herein. Where a specific range of values is provided, it is understood that each intervening value, to the tenth or less of the unit of the lower limit between the upper and lower limit of that range and any other stated or intervening value in that stated range of sub range thereof, is included herein unless the context clearly dictates otherwise. All subranges are also included. The upper and lower limits of these smaller ranges are also included therein, subject to any specifically and expressly excluded limit in the stated range.

It should be noted that some of the terms used herein are relative terms. For example, the terms, "upper" and "lower," "above" and "below" are relative to each other in location, *i.e.*, an upper component is located at a higher elevation than a lower component in the indicated or depicted orientation, but these terms can change if the orientation is flipped.

The terms, "horizontal" and "vertical" are used to indicate direction relative to an absolute reference, *i.e.*, ground level. However, these terms should not be construed to require structure to be absolutely parallel or absolutely perpendicular to each other. For example, a first vertical structure and a second vertical structure are not necessarily parallel to each other.

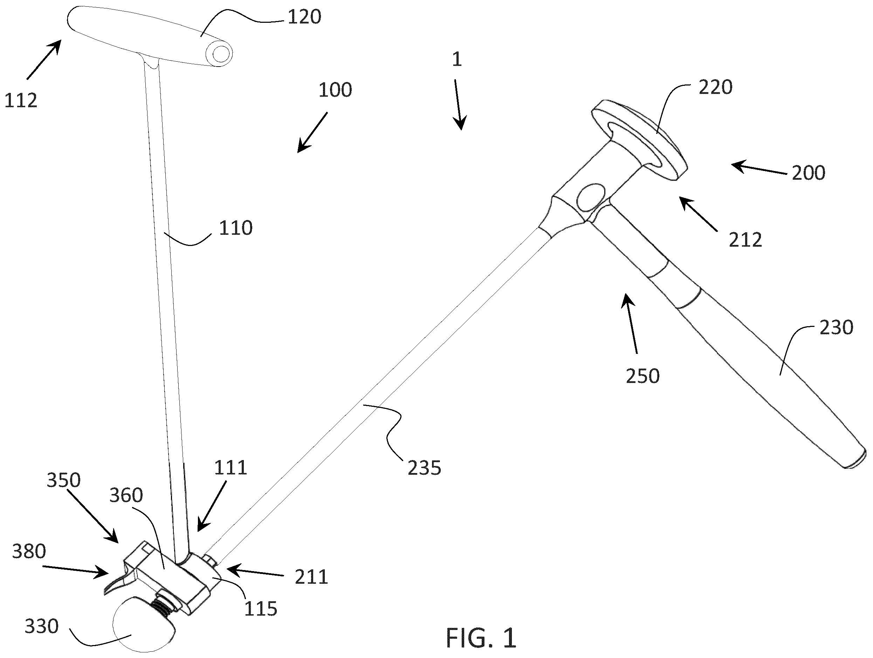

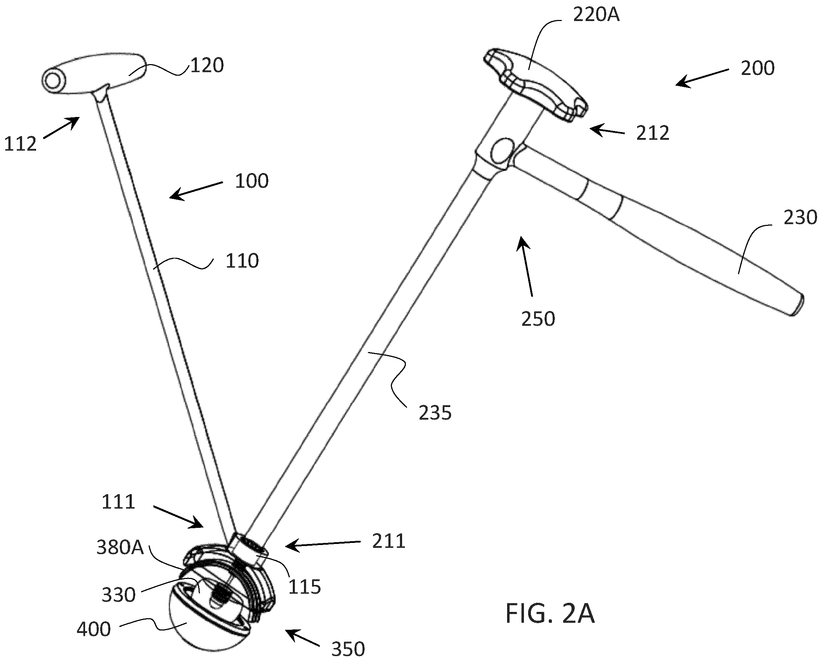

As indicated in **FIGS. 1** - **7****,** exemplary embodiments of the detachable acetabular cup remover instrument assembly **1** are configured for use in removing a primary acetabular cup or combination of cup and shell **400 (****FIG. 2A****)** (collectively, "primary cup") in a revision hip procedure. Although the instrument **1** is described herein for removal of primary cups, it can be used to remove revision cups when revision cups are present.

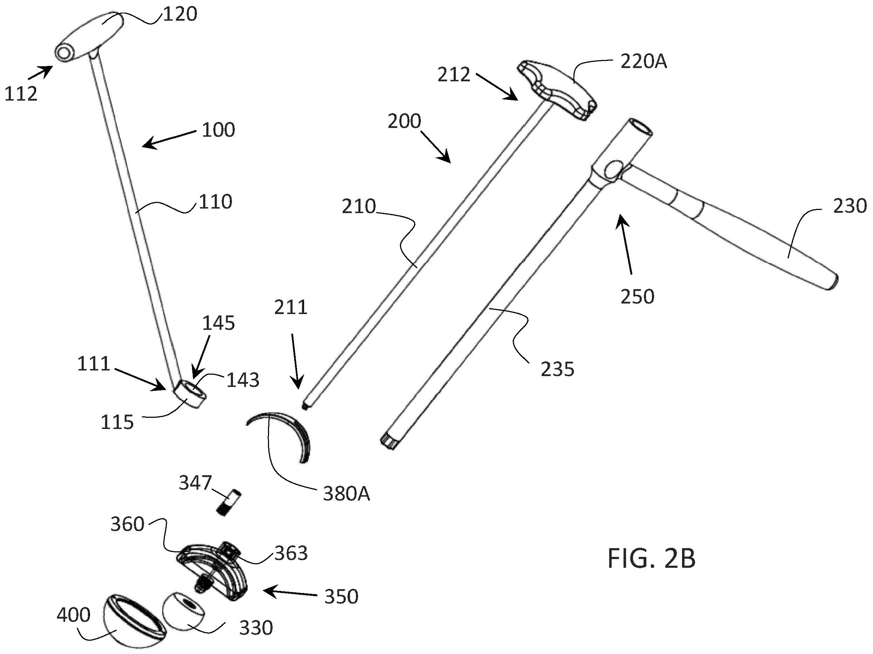

The exemplary cup remover instrument assemblies **1** described herein have an assembled configuration (see *e.g.*, **FIG. 2A****),** a disassembled configuration (see **FIG. 2B****),** and partially assembled configurations.

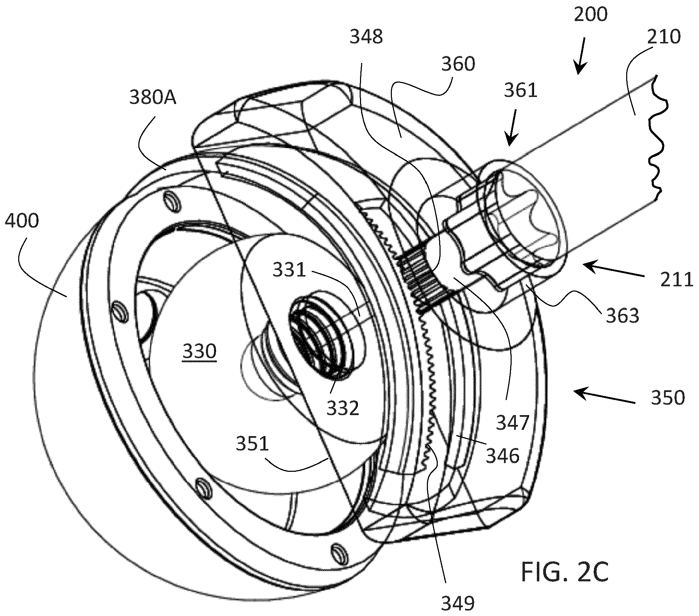

The exemplary cup remover instrument assemblies **1** include, generally, a positioning member **100** comprising a longitudinal body **110,** a leading end **111,** and a trailing end **112.** The longitudinal body **110** extends between the leading end **111** and the trailing end **112.** The positioning member engages an orientation bearing **115** having an inner surface **143 (****FIG. 2B****)** defining a through hole **145 (****FIG. 2B****).** A cutting assembly **350** having a mating portion **363 (****FIG. 2B****)** is configured to be rotatably disposed within the through hole **145** of the orientation bearing **115.** Referring briefly to **FIG. 2C****,** the mating portion **363** can comprise an outer circumferential surface. Referring briefly to **FIGs. 2A** and **2B****,** the inner surface **143** of the orientation is concentrically and closely disposed around the outer circumferential surface of the mating portion **363** when the cup remove instrument **1** is in the assembled configuration. In this manner, the mating portion **363** can be said to be "configured to be rotatably disposed within the through hole **145** of the orientation bearing **115."**

The cutting assembly **350** comprises a cutting blade **380.** A drive member **200** having a drive member body **210 (****FIG. 2B****)** extends between a drive member leading end **211** and a drive member trailing end **212.** The leading end **211** of the drive member **200** engages the cutting assembly **350** to rotatably drive the cutting assembly **350** to cut around the acetabular cup implant **400** and thus detach the acetabular cup implant **400** from a patient's natural acetabulum. It is contemplated that the positioning member **100,** drive member **200** orientation bearing **115,** and cutting assembly **350** can be made from medical grade stainless steel, titanium, or any other biocompatible material having similar strength and durability properties.

Various configurations of cutting arrangements and drive mechanisms for operating the various cutting arrangements will be described herein. In certain exemplary embodiments, the positioning member **100** is sized and configured to operate in or through a main incision, while the drive member **200** is sized and configured to operate in or through a portal incision.

### Fixed Blade

In the embodiment of **FIG. 1****,** the exemplary cup remover assembly **1** includes a single fixed cutting blade **380** that can be rotated around the outer edge of an acetabular cup implant **400** (see **FIG. 2A****).** The positioning member **100** includes a longitudinal body **110,** such as a main shaft or an extension portion extending between a leading or lower end **111** and a trailing or upper end **112.** An orientation bearing **115** is affixed to or can be integral with the leading end **111.** In exemplary embodiments, the orientation bearing **115** is a ring member configured to rotatably receive and support a cutting assembly **350.** In embodiments, a hand grip **120** is provided on or adjacent to the trailing end **112** of the longitudinal body **110.** The hand grip **120** can be in the form of a T-handle, as shown in **FIG. 1****.** The hand grip **120** is configured for use in inserting the positioning member **100** and the cutting assembly **350** into a main incision that is aligned with the patient's hip joint. A partially engaged configuration, in which the positioning member **100** engages the cutting assembly **350** can be used to position the cutting assembly **350** over the patient's acetabulum and to seat a femoral head bearing **330** into the acetabular cup implant **400** that is to be removed.

A cutting assembly **350** is rotatably attached adjacent to the leading end **111** of the positioning member **100** via the orientation bearing **115.** In the depicted embodiment, the cutting assembly **350** comprises a blade support portion **360** rotatably connected to the orientation bearing **115** in an assembled or partially assembled configuration, a cutting blade **380** extending distally from the blade support portion **360,** and a femoral head bearing **330.** The femoral head bearing **330** is positioned below the blade support portion **360.** The femoral head bearing **330** is typically generally hemispherical and is typically sized for use in orienting the cup remover assembly **1** in the primary acetabular cup **400** (see **FIG. 2A****).** The femoral head bearing **330** may be rotatably connected below the blade support portion **360** such that the blade support portion **360** rotates around and independently of the femoral head bearing **330.** As shown in **FIG. 1****,** the cutting blade **380** typically extends distally from the blade support portion **360** and has a curved or arcuate cross section generally matching an outer diameter of the acetabular cup implant **400** to be removed. The cutting blade **380** is positioned a selected distance from an outer diameter of the femoral head bearing **330** such that the cutting blade **380** cuts bone or bone cement adjacent an outer diameter of the acetabular cup implant **400** to be removed. A driver seat **361 (****FIG. 2C****)** extension of the blade support portion **360** can be rotatably positioned in the orientation bearing **115** for use in rotating the blade support portion **360** and the cutting blade **380,** as described herein.

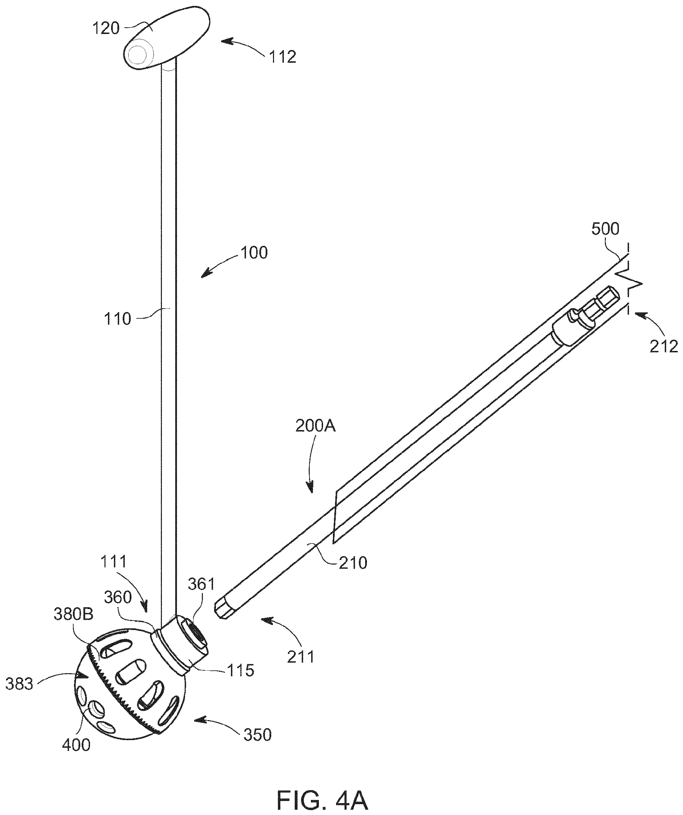

A separate drive member **200** is provided for use in rotating the cutting assembly **350.** The drive member **200** (see also **FIG. 2B****)** has a drive member body **210** extending between a drive member leading or lower end **211** and a drive member trailing or upper end **212.** In the embodiment of **FIG. 1****,** the drive member body **210** has been inserted through a portal guide assembly **250.** The portal guide assembly **250** can comprise a portal guide **235** that can be inserted through a portal incision in the patient's leg if desired. In other exemplary embodiments, the portal guide assembly **250** can be inserted through a cannula **500 (****FIG. 4A****).** The portal guide assembly **250** may optionally include a drive handle **230** extending from the portal guide **235** adjacent to the drive member trailing end **212** in the assembled configuration. The drive handle **230** is desirably positioned and configured for use in rotating the drive member **200** to rotate the blade support portion **360** and the attached cutting blade **380** via the drive seat **361** when engaged. The drive handle **230** engages the drive member trailing end **212** in any manner appreciable by those having ordinary skill in the art, but desirably by a mechanical projection-receiver engagement mechanism as discussed below with reference to the leading end **211** of the drive member **200.** In this manner, the drive handle **230** can be said to be "configured to" engage the drive member trailing end **212** to thereby rotate the drive member body **210** and the drive member leading end **211.**

The leading end **211** of the drive member **200** is configured to engage the drive seat **361** of the blade support member **360,** such as via a male hex on the leading end **211** and a female hex on the drive seat **361,** or vice versa. All projections and closely fitting receiver engagement mechanisms known to those having ordinary skill in the art are considered to be within the scope of this disclosure. In this manner, the drive member leading end **211** can be said to be "configured to engage the cutting assembly **350** to rotatably drive the cutting assembly **350."** A handle **220** is provided on or at the trailing end **212** of the drive member **200** for use in holding, inserting, removing, and manipulating the drive member **200** in the portal guide assembly **250** and into engagement with the drive seat **361** disposed within the orientation bearing **115** of the positioning member **100.**

Using the configuration described herein, a surgeon can selectively position the positioning member **100** in a main incision and the drive member **200** in a portal incision and selectively rotate the drive member **200** to rotate the cutting blade **380** around the acetabular cup implant **400** that is to be removed.

In order to accommodate and remove diverse sizes of acetabular cup implants (*i.e*., hip cups), the fixed cutting blade **380** is configured to be removable such that different sized cutting blades **380** can be selected for use in removing different sizes of cups. In exemplary embodiments, multiple sizes of blade support portions **360,** cutting blades **380,** and femoral head bearings **330** may be provided, such as in the form of an instrument kit arranged in a surgical tray.

For example, it is contemplated that the blade support portion **360** has a length dimension that can be sized along with the cutting blade **380** to place the cutting blade **380** just outside of the outer diameter of the acetabular cup implant **400** to be removed. The size of the acetabular cup implant **400** is desirably known prior to surgery (*e.g*., through patient records or pre-operative radiographs) such that the blade support portion **360** and the cutting blade **380** can be sized to accommodate the removal of the acetabular cup implant **400.** However, it is also contemplated that more than one blade support portion **360** or cutting blade **380** having different size dimensions can be provided at the time of surgery to provide the surgeon with options when the surgeon encounters the actual acetabular cup implant **400** during the procedure.

It is further contemplated that fixed cutting blades **380** having different blade lengths can be provided. A surgeon may use a shorter cutting blade **380** to initiate the removal of the acetabular cup implant **400.** For example, a shorter cutter blade **380** may create a useful opening between the natural acetabulum and the acetabular cup implant **400** around the outer circumference of the acetabular cup implant **400.** A longer cutting blade **380** placed within the initial opening can be used to extend the opening between the acetabular cup implant **400** and the natural acetabulum to thereby separate the acetabular cup implant **400** from the natural acetabulum.

Differently sized femoral head bearings **330** may be used to seat various sized acetabular cup implants **400.** In exemplary embodiments, the various sizes are integral with a respective positioning member **100.** In other embodiments, the components may be removable from and detachable from a positioning member **100** so as to reduce instrument inventory.

### Extendable Blade

In the embodiment of **FIGs. 2A - C,** the cup remover assembly **1** is similar to the exemplary embodiment described with reference to **FIG. 1****.** However, the cutting blade **380** is selectively extendable from the blade support portion **360** (see **FIG. 2C****).** In the embodiment of **FIGs. 2A- C,** this is accomplished by providing a hollow portal guide **235** that houses the drive member body **210.** An adjustment handle **220A** is provided on the trailing end **212,** rather than the fixed handle **220** of the embodiment of **FIG. 1****.** The selectively extendable cutting blade **380A** is operably connected to the drive member **200 (****FIG. 2B****)** such that rotation of the adjustment handle **220A,** *e.g.,* as in a clockwise direction, selectively extends the cutting blade **380A.** Once a desired cutting blade length has been set, the surgeon can disengage the drive member **200** from the cutting assembly **350** to define a partially engaged position (*i.e.*, the orientation bearing **115** of the positioning member **100** is still disposed around the mating portion **363** of the cutting assembly **350).** The surgeon may then use the handle **120** of the positioning member **100** to selectively move the cutting assembly, and by extension, the selectively extendable cutting blade **380A** around the outer surface of the acetabular cup implant **400** to thereby unseat the acetabular cup implant **400** from the patient's natural acetabulum. The length of the selectively extendable cutting blade **380A** can be adjusted as needed relative to the bottom **351** of the cutting assembly support **360** during the procedure.

**FIG. 2C** provides a close up view of an exemplary operable connection of the cutting blade **380** to the drive member **200.** In the assembled configuration, the leading end **211** of the drive member **200** comprises a male keyed shape, such as a hex shape. The male keyed shape is disposed in and engages the sides of a complementary keyed shape, such as a female hex shape on the drive seat **361.** In this manner, the drive seat member **361** can be said to have a proximal end that has a keyed interface that is "configured to mate" with a complementary keyed inface of the drive member leading end **211.**

The drive seat **361** is disposed within the mating portion **363** of the cutting assembly **350.** In an assembled configuration, the mating portion **363** is disposed within the hole **145** of the preferably annular inner surface **143** of the orientation bearing **115.** In this manner, the mating portion **363** can be said to be "configured to be rotatably disposed" within the hole **145** of the orientation bearing **115.** While an annular inner surface **143** comprises a preferred embodiment, any mechanical engagement that permits the mating portion **363** to be positioned relative to the movement of an engaged positioning member **100** is considered to be within the scope of this disclosure.

The drive seat **361** is engaged to and shares a rotational axis with a spline shaft **347** comprising distal spline teeth **348.** The distal spline teeth **348** in turn selectively engage geared teeth **349** of the extendable cutting blade **380.** The geared teeth **349** can be oppositely disposed from a non-geared blade support side **346.** In this manner, rotation of the adjustment handle **220A,** *e.g.,* as in a clockwise direction, selectively extends the cutting blade **380A** relative to the bottom **351** of the blade support potion **360.** The foregoing description is one example of how a blade support portion **360** can have a drive seat that is "configured to communicate with" the cutting blade **380.**

In certain exemplary embodiments, a sleeve may be closely disposed between the spline shaft **347** and an inner wall of the drive seat **361.** Such a sleeve can provide a frictional force that prevents rotation of the spline shaft **347** when said frictional force is not overcome by the rotational movement of the engaged drive member **200.** In this manner the sleeve can lock the spline shaft **347,** and by extension, the selectively extendable cutting blade **380A** at the desired location, which can correspond to the desired length of the selectively extendable cutting blade **380A** relative to the bottom **351** of the cutting assembly support **360.**

It will be appreciated that a tangential protrusion and receiver locking mechanism or similar mechanical or electromechanical locking structure that prevents rotational movement of the spline shaft **347** when the engaged drive member **200** is not spinning is within the scope of this disclosure. The spline shaft **347** may be generally cylindrically and annularly disposed around a fixed femoral head bearing support **331** in exemplary embodiments. The fixed femoral head bearing support **331** extends beyond the spline shaft **347** and desirably supports a femoral head engagement mechanism **332.** The femoral head engagement mechanism is configured to detachably engage a femoral hear bearing **330.** Exemplary femoral head engagement mechanisms **332** can comprise a screw thread, a ball bearing in a complementary socket, a clamp, a protrusion, a recess, and other known means to selectively engage and disengage one component from another mechanically.

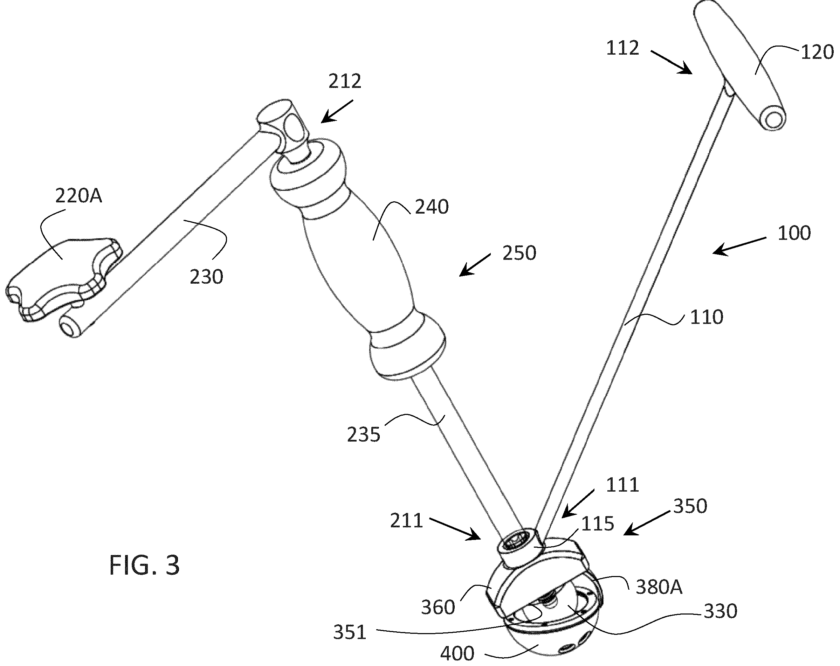

**FIG. 3** shows an alternative embodiment of a selectively extendable cutting blade **380A** in which the extension of the blade **380** is timed to the rotation of the drive handle **230.** In this embodiment, the blade **380** extends a small amount relative to the bottom **351** of the cutting assembly support **360** with each rotation of the drive handle **230.** In this manner, the cutting blade **380** cuts deeper with each rotation. In the embodiment of **FIG. 3****,** this is accomplished by providing the drive member **200** (see **FIG. 2B****)** through the portal guide **235** of the portal guide assembly **250.** The portal guide assembly **250** may optionally further comprise a fixed handle **240** on the upper portion of the portal guide **235** for use in holding and manipulating the dive member **200.** As with the embodiments depicted in **FIG. 1** and **FIGs. 2A- C,** the portal guide assembly **250** can be inserted through a portal incision in the patient's leg if desired. In other exemplary embodiments, the portal guide assembly **250** can be inserted through a cannula **500 (****FIG. 4A****).** The cutting blade **380** is operably connected to the internal drive member **200** such that rotation of the drive handle **230,** *e.g.*, as in a clockwise direction, selectively extends the cutting blade **380.** In the depicted embodiment, the cutting blade **380** is operatively connected to the drive member **200** and can be selectively lockable in the same way as described with reference to **FIG. 2C** above.

### Powered Blade

In the embodiment of **FIG. 4A****,** the detachable cup remover assembly **1** of the present disclosure includes a powered hemispherical cutting blade **380B.** In the embodiment of **FIG. 4A****,** the hemispherical cutting blade **380B** has a hollow hemispherical interior and an annular rim on a lower end thereof. The hemispherical cutting blade **380B** is provided with a cutting edge (see **383)** along the annular bottom rim, such as a serrated edge or serrations in the manner of a saw blade. The hemispherical cutting blade **380B** desirably comprises one or more evacuation holes **385** extending through a blade body of the hemispherical cutting blade **380B.** The evacuation holes **385** permit removed bone cement, bone, marrow, or other tissue to exit the drilling area without unduly interfering with the cutting process.

In embodiments, various sizes of hemispherical blades **380B** are provided such that the blade **380B** can be selectively matched to the size of a primary or revision acetabular cup implant **400** to be removed. The annular rim is selectively sized to extend a cutting end of the hemispherical cutting blade **380B** around the fixed primary or revision acetabular cup implant **400.** The hemispherical cutting blade **380B** can be power driven by the drive member **200,** which in this embodiment takes the form of a portal drive shaft **200A.** A trailing end **212** of the portal drive shaft **200A** is provided with a standard configuration for engagement by an electric drill or other power drive mechanism, such as an engagement flat or flats.

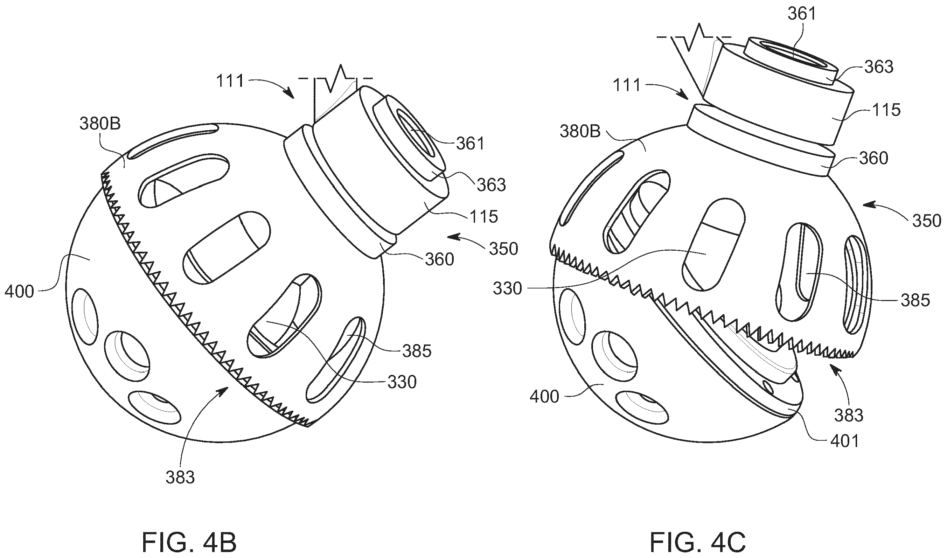

As indicated in **FIGs. 4B** and **4C** (see also **FIG. 4A****),** the T-handle **120** of the positioning member **100** can be used to move the hemispherical blade **380B** around the outer diameter of the primary or revision acetabular cup implant **400** during cutting. **FIG. 4B** shows the hemispherical cutting blade **380B** oriented directly over the acetabular cup implant **400** in a starting position. **FIG. 4C** shows the hemispherical cutting blade **380B** rotated around a side of the acetabular cup implant **400** to assist in cutting deeper areas of the adjacent bone or bone cement.

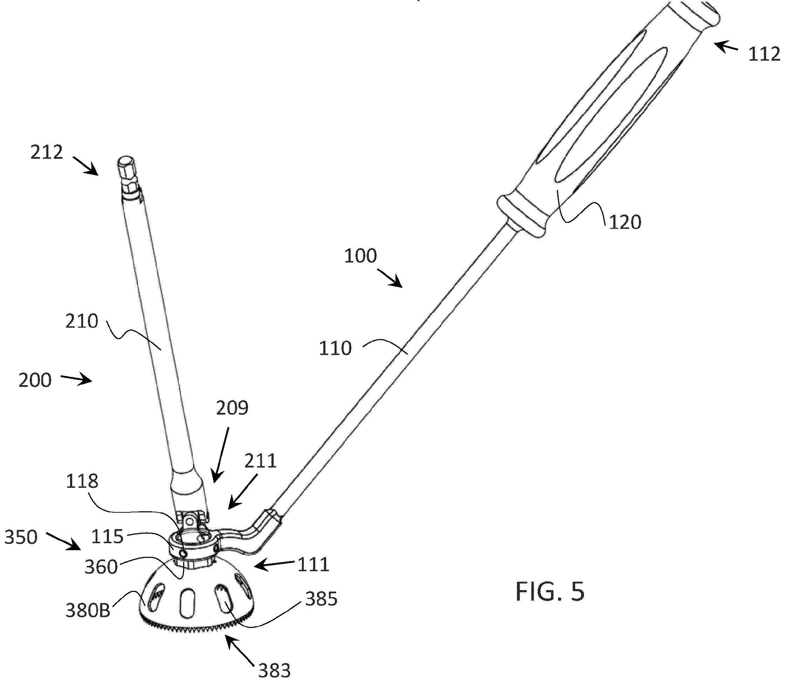

The powered hemispherical cutting blade **380B,** while powerful, presents several design limitations. Depending on the size of the inner diameter of the acetabular cup implant **400,** a liner insert **401** may be needed in order to maintain the hemispherical cutting blade **380B** in a concentric orientation with the outer diameter of the primary or revision acetabular cup implant **400.** Liner inserts **401** can be provided to accommodate various sizes of acetabular cup implants **400** and hemispherical cutting blades **380B.** Additionally, it is contemplated that the use of a portal drive shaft **200A** having a ball end, universal joint (see **209,** **FIG. 5****)** or a gimbled connection (not shown) will improve maneuverability of any of the blades **380** considered to be within the scope of this disclosure around the acetabular cup insert **400** during cutting.

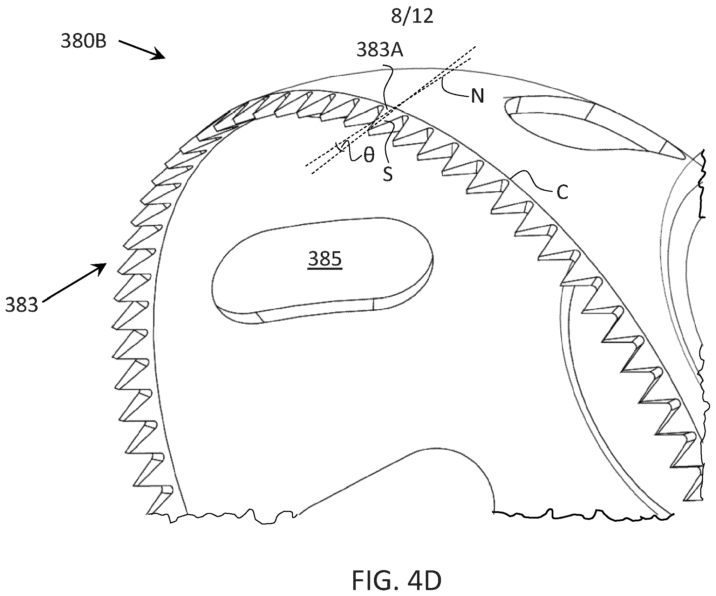

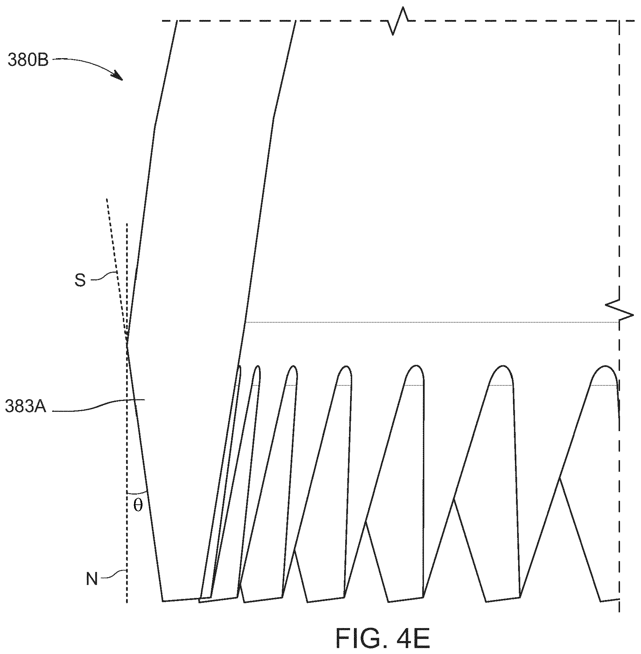

As seen more clearly in **FIGs. 4D** and **4E****,** the serrated edge comprises serrated teeth **383** extending from a circumference **C** of the hemispherical cutting blade **380B.** In the depicted embodiment, the serrated teeth **383** have an outer surface angle **θ** defined by the intersection between a normal line **N** to the circumference **C** of the hemispherical cutting blade **380B** and the plane defined by an outer surface **S** of a serrated tooth **383A** of the serrated teeth **383.** In certain exemplary embodiments, the outer surface angle **θ** is desirably less than 10 degrees. In other exemplary embodiments, the outer surface angle **θ** can be a compound angle comprising the sum of multiple angles, including for example, combinations of outer surface angles and normal angles. In still other exemplary embodiments, the outer surface **S** of the serrated tooth **383A** can comprise multiple outer surface angles **θ** wherein the second and any subsequent angles disposed below an initial angle, the initial angle being disposed closest to the circumference **C,** are greater than the preceding outer surface angle **θ** relative to the normal line **N.** In still other exemplary embodiments, the serrated teeth **383** may have a portion that is coextensive with the normal line **N** and be angled relative to a line that is coplanar with the normal plane to define a normal angle. In still other exemplary embodiments, outer surface **S** of the serrated tooth **383A** can comprise multiple normal angles wherein the second and any subsequent angles are greater than the preceding normal angle relative to the line that is coplanar with the normal plane. Combinations of normal angles and outer surface angles **θ** are considered to be within the scope of this disclosure. Any arrangement of teeth **383** that closely follows the outer curvature of the acetabular cup implant **400** in in such manner that pulls the acetabular cup implant **400** toward the cutting assembly support **360** during operation is considered to be within the scope of this disclosure.

Without being bound by theory, it is contemplated that a hemispherical cutting blade **380B** having serrated teeth **383** with an outer surface angle **θ** of desirably less than 10 degrees applies a pulling force on the acetabular cup implant **400** during active use. That is, the hemispherical cutting blade **380B** with the serrated teeth **383** described herein may facilitate the removal of the acetabular cup implant **400** by drawing the acetabular cup implant **400** toward the bottom **351** of the cutting assembly support **360** during normal use. As a result, it is contemplated that surgeons may be able to spend less time removing prior-implanted acetabular cup implants **400,** to thereby reduce procedure time and its ancillary risks.

The components of the cup remover instrument assembly **1,** including different sizes of components, will typically be arranged in a convenient format, such as in a surgical tray or case, in the manner of a kit. However, the kit components do not have to be packaged or delivered together, provided that they are assembled or collected together in the operating room for use at the time of surgery.

**FIG. 5** is a side perspective view of another exemplary embodiment of the present disclosure in which the orientation bearing **115** is directly integrally engaged to the leading end **111** of the positioning member **100** and wherein the annular inner surface **143** of the orientation bearing **115** further comprises ball bearings **118** disposed within the orientation bearing **115** and extending into the hole **145** of the orientation bearing **115.** In an assembled configuration, or in a partially assembled configuration in which the cutting assembly **360** is rotatably disposed within the hole **145** of the orientation bearing **115,** the ball bearings **118** can be closely disposed adjacent to a track in the cutting assembly support **360** to facilitate the rotational movement of the cutting assembly support **360** relative to the orientation bearing **115.**

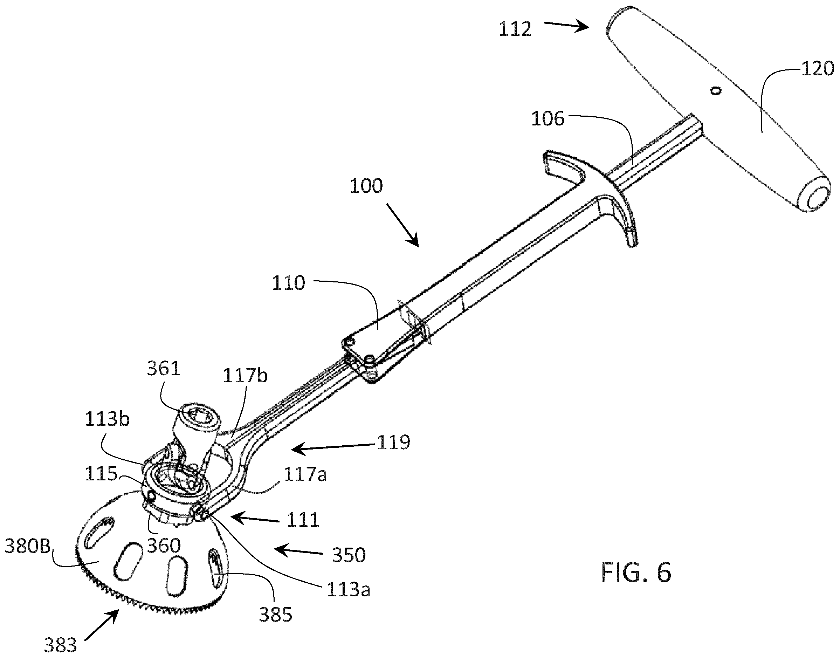

In the depicted embodiment, the drive member **200** comprises a universal joint **209** at the drive member leading end **211.** In practice, it is contemplated that the drive member **200,** which is a portal drive shaft as in the depicted embodiment, may be inserted through a portal incision in the patient's leg that aligns with the exposed surgical area. The exposed surgical area can be defined by a main surgical incision positioned over the patient's joint capsule relative to the surgeon's point of view. The surgeon may place the cutting assembly **350** comprising a cutting assembly support **360** and a hemispherical cutting blade **380** into the main incision. The cutting assembly **350** may be rotatably engaged to the orientation bearing **115,** which is in turn directly or indirectly engaged to the positioning member **100** while inserted into the main incision. The drive member **200** may be inserted into the portal incision. A cannula **500** or other tube may be inserted through the portal incision prior to the insertion of the drive member **200.** A cannula **500** or other liner can be desirable to preserve patient tissue surrounding the portal incision in view of the rotational movement of the drive member **200.** Once both the cutting assembly **350** and the drive member leading end **211** are present in the main surgical area, the surgeon then engages the drive member leading end **211** to the drive seat **361** of the cutting assembly **350.** In the depicted embodiment, the drive seat **361** of the cutting assembly **350** is disposed on a trailing end (see 111) of a universal joint **209** (see **FIG. 6****).** The depicted universal joint **209** can be part of the cutting assembly support **360.** The cutting assembly support **360** in turn supports the cutting blade **380,** which is a hemispherical cutting blade **380B** in the depicted embodiment.

Without being bound by theory, it is contemplated that the presence of the universal joint **209,** or a similar omni-directional joint, permits the surgeon to use the positioning member **100** to adjust the position of the hemispherical cutting blade **380B** in an arcuate manner relative to the acetabular cup implant **400** (see **FIGs. 4B** and **4C****)** while maintaining the rotational movement of the hemispherical cutting blade **280B** around a center rotational axis **R** via the drive member **200.** Such an embodiment may permit surgeons to use a portal incision and main incision minimally invasive technique to remove the prior-installed acetabular cup implant **400** in a revision hip arthroplasty procedure. It is contemplated that the speed at which this may be accomplished could reduce the overall time that the patient is under anesthesia, reduce procedure time, and therefore the risk of infection, while maintaining the enhanced recovery time associated with minimally invasive procedures.

Although a universal joint **209** is depicted, all joints that permit variations in alignment or distance between the drive member **200** and the cutting assembly support **360** are considered to be within the scope of this disclosure, including for example: jaw couplings, rag joints, splined joints, a balled end and gimbled connection, and prismatic joints.

**FIG. 6** is a side perspective view of an exemplary cup remover instrument assembly **1** that is similar to the embodiment described with reference to **FIG. 5****,** except that the positioning member **100** further comprises an articulating clamp **119** at the leading end **111.** The articulating clamp **119** comprises a first arm **117a** and a second arm **117b** oppositely and distally disposed to the first arm **117a.** Pins **113a, 113b** extend through aligned pin holes in the orientation bearing **115** and in the first arm **117a** and the second arm **117b** to indirectly engage the orientation bearing **115** to the leading end **111** of the positioning member **100.** In this manner, the positioning member **100** can be said to "indirectly engage" the orientation bearing **115.** In the depicted embodiment, the **T** handle **120** of the positioning member **100** engages a trailing end of a follower **106.** The surgeon can apply a counter force when pressing the T handle **120** and the follower **106** toward the leading end **111** of the positioning member **100.** The follower **106** extends generally within the positioning member **100** along the length of the positioning member **100** until the follower **106** reaches the articulating clamp **119.** A surgeon can push the follower **106** toward the leading end **111** to extend the first arm **117a** and the second arm **117b** away from one another to release the orientation bearing **115.**

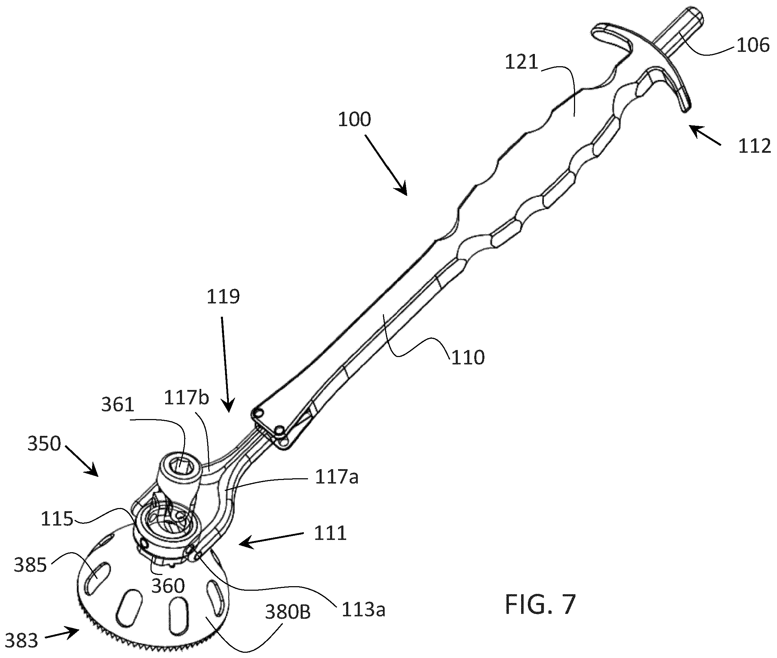

The exemplary embodiment of **FIG. 7** is similar to the exemplary embodiment of **FIG. 6** except that the body **110** of the positioning member **100** comprises a grip **121** that extends generally lengthwise along the length of the body **110** of the positioning member **100.** The follower **106** may engage a T handle **120** as shown in **FIG. 6****,** a different type of handle, or no handle at all.

The instruments that comprise the exemplary cup remover instrument assembly **1** can be provided in the form of a kit. The components of the kit are preferably arranged in a convenient format, such as in a surgical tray or case. However, the kit components do not have to be packaged or delivered together, provided that they are assembled or collected together in the operating room for use at the time of surgery. An exemplary kit can include any suitable embodiment of a cup remove instrument assembly **1,** variations of the cup remove instrument assembly **1** described herein, and any other cup remove instrument assembly **1** according to an embodiment. While it is contemplated that an exemplary kit may further include one or more cutting assemblies **350,** one or cutting blades **380,** one or more types of cutting blades **380,** (*e.g*., selectively extendable cutting blades **380A,** or hemispherical cutting blades **380B),** one or more drive members **200,** one or more cutting assembly supports **360,** one or more orientation bearings **115,** and one or more positioning members **100,** it will be appreciated that certain kits may lack some or all of these elements. Any suitable embodiment of a cutting assembly **350,** variations of the cutting assemblies **350** described herein, and any other cutting assemblies **350** according to an embodiment are considered to be within the scope of this disclosure. Any suitable embodiment of a cutting blade **380,** variations of the cutting blades **380** described herein, and any other cutting blade **380** according to an embodiment are considered to be within the scope of this disclosure. Any suitable embodiment of a drive member **200,** variations of the drive members **200** described herein, and any other drive member **200** according to an embodiment are considered to be within the scope of this disclosure. Any suitable embodiment of a cutting assembly support **360,** variations of the cutting assembly supports **360** described herein, and any other cutting assembly support **360** according to an embodiment are considered to be within the scope of this disclosure. Any suitable embodiment of an orientation bearing **115,** variations of the orientation bearings **115** described herein, and any other orientation bearing **115** according to an embodiment are considered to be within the scope of this disclosure. Any suitable embodiment of a positioning member **100,** variations of the positioning members **100** described herein, and any other positioning member **100** according to an embodiment are considered to be within the scope of this disclosure.

Selection of a suitable number or type of cup remover instrument assembly **1,** cutting assembly **350,** cutting blade **380,** drive member **200,** cutting assembly support **360,** orientation bearing **115,** and positioning member **100** to include in a kit according to a particular embodiment can be based on various considerations, such as the procedure intended to be performed using the components included in the kit.

### Methods of Use

In operation, the cup remover **1** is configured for use in a two-incision hip procedure, such as those discussed in the background section. In two-incision hip procedures, a main incision provides access to the hip joint, while a nearby portal incision communicates with the hip joint. Relatively large instruments are inserted into the hip joint through the main incision, while the portal incision is used to drive or otherwise operate the instruments located in the hip joint. Two-incision hip procedures have traditionally been used for primary hip procedures. However, the cup remover **1** of the invention is designed for use in two-incision revision procedures. As discussed above, the positioning member **100** is sized and configured to operate in or through a main incision, while the drive member **200** is sized and configured to operate in or through a portal incision. The positioning member can be manipulated in the main incision via the hand grip **120** and the upper portion of the extension portion **120.** The configuration of the drive member **200** allows it to be inserted through a portal incision and operated via the handle **220** and drive handle **230** portions. If a cannula **500** is used in the portal incision, a portal drive shaft **200** can be inserted into the cannula **500** and used to drive the cutting blade **380.**

An exemplary medical device assembly comprises: an orientation bearing having an inner surface defining a hole, a positioning member engaged to the orientation bearing, the positioning member having a longitudinal body extending between a leading end and a trailing end, a cutting assembly having a mating portion configured to be rotatably disposed within the hole of the orientation bearing, wherein the cutting assembly comprises a cutting blade, and a drive member having a drive member body extending between a drive member leading end and a drive member trailing end, wherein the drive member leading end is configured to engage the cutting assembly to rotatably drive the cutting assembly.

In an exemplary embodiment, the medical device assembly can further comprise a femoral head bearing disposed below a blade support portion.

In an exemplary embodiment, the cutting assembly can further comprise: a blade support portion rotatably connected to the cutting assembly support. In yet a further exemplary embodiment, the blade support portion is a drive seat configured to communicate with the cutting blade, and the cutting blade extends distally from the blade support portion, and the leading end of the drive member is configured to engage the drive seat.

In an exemplary embodiment, the orientation bearing is integrally engaged to the leading end of the positioning member. In another exemplary embodiment, the orientation bearing is indirectly engaged to the leading end of the positioning member.

In an exemplary embodiment, the cutting blade is a hemispherical cutting blade. In such an embodiment, the hemispherical cutting blade may further comprise serrated teeth extending from a distal circumference of the hemispherical cutting blade. In such an embodiment, the serrated teeth may further have an outer surface angle defined by an angle between a normal line to the distal circumference of the hemispherical cutting blade and an outer surface of a serrated tooth of the serrated teeth. In such an embodiment, the outer surface angle may be less than 10 degrees.

In an exemplary embodiment comprising a hemispherical cutting blade, the hemispherical cutting blade further defines an evacuation hole extending through a blade body of the hemispherical cutting blade. In such an exemplary embodiment, the hemispherical cutting blade may further define a plurality of evacuation holes extending through a blade body of the hemispherical cutting blade.

In an exemplary embodiment, the cutting assembly further comprises a cutting assembly support, and wherein the cutting assembly support has the mating portion configured to be rotatably disposed within the hole of the orientation bearing. In such an exemplary embodiment comprising a cutting assembly support, the medical device assembly may further comprise a drive seat member disposed between the cutting assembly support and the drive member in an assembled configuration. In such an exemplary embodiment comprising a cutting assembly support, the cutting assembly support may have a drive seat member configured to communicate with the cutting blade, the cutting blade can extend distally from the cutting assembly support, and the leading end of the drive member can be configured to engage the drive seat.

In an exemplary embodiment comprising a drive seat member, the drive seat member may comprise a proximal end having a keyed interface configured to mate with a complementary keyed inface of the drive member leading end.

In an exemplary embodiment having a keyed interface, the cutting blade is a curved cutting blade, wherein a spline shaft extends distally from the keyed interface, and wherein geared teeth on a distal end of the spline shaft engage complementary geared teeth of the curved cutting blade disposed in the cutting assembly support such that a rotational movement of the spline shaft translates into an arcuate movement of the curved cutting blade.

In an exemplary embodiment, the positioning member may further comprise an articulating clamp at the leading end, the articulating clamp may comprise a first arm and a second arm, and pins may extend through aligned pin holes in the orientation bearing and the first arm and the second arm to indirectly engage the orientation bearing to the positioning member.

In an exemplary embodiment, the medical device assembly may further comprise a handle disposed at the trailing end of the positioning member.

In an exemplary embodiment, the medical device assembly may further comprise a drive handle configured to engage the drive member trailing end to thereby rotate the drive member body and the drive member leading end.

In an exemplary embodiment, the cutting assembly is rotatably engaged to the cutting assembly support.

In an exemplary embodiment, the medical device assembly can further comprise a cannula, wherein the drive member is disposed within the cannula.

An exemplary medical device assembly can comprise: a cutting assembly support having a drive seat configured to communicate with a curved cutting blade, the curved cutting blade extending distally from the cutting assembly support, and a drive member having a drive member body extending between a drive member leading end and a drive member trailing end, wherein the drive member is configured to engage the drive seat to arcuately extend the curved cutting blade.

An exemplary medical device assembly can comprise: an orientation bearing having an inner surface defining a hole; a positioning member engaged to the orientation bearing, the positioning member having a longitudinal body extending between a leading end and a trailing end, a cutting assembly support having a mating portion configured to be rotatably disposed within the hole of the orientation bearing, a cutting assembly engaged to the cutting assembly support, wherein the cutting assembly comprises a cutting blade, and, a drive member having a drive member body extending between a drive member leading end and a drive member trailing end, wherein the drive member trailing end is configured to engage the cutting assembly support to rotatably drive the cutting assembly.

## Claims

1. A medical device assembly (1) comprising:
an orientation bearing (115) having an inner surface (143) defining a hole (145);
a positioning member (100) engaged to the orientation bearing (115), the positioning member (100) having a longitudinal body (110) extending between a leading end (111) and a trailing end (112);
a cutting assembly (350), wherein the cutting assembly (350) comprises a cutting blade (380) and a cutting assembly support (360), and wherein the cutting assembly support (360) has a mating portion (363) configured to be rotatably disposed within the hole (145) of the orientation bearing (115);
a drive member (200) having a drive member body (210) extending between a drive member leading end (211) and a drive member trailing end (212), wherein the drive member leading end (211) is configured to engage the cutting assembly (350) to rotatably drive the cutting assembly (350); and
a drive seat (361) disposed between the cutting assembly support (360) and the drive member (200) in an assembled configuration, wherein the drive seat (361) is configured to communicate with the cutting blade (380), the cutting blade (380) extending distally from the cutting assembly support (360), wherein the drive member (200) is configured to engage the drive seat (361), and wherein the drive seat (361) comprises a proximal end having a keyed interface configured to mate with a complementary keyed interface of the drive member leading end (211);
**characterized in that**:
the cutting blade (380) is a curved cutting blade (380A), wherein a spline shaft (347) extends distally from the keyed interface of the drive seat (361), and wherein distal spline teeth (348) on a distal end of the spline shaft (347) engage complementary geared teeth (349) of the curved cutting blade (380A) disposed in the cutting assembly support (360) such that a rotational movement of the spline shaft (347) translates into an arcuate movement of the curved cutting blade (380A).

2. The medical device assembly (1) of claim 1, further comprising a femoral head bearing (330) disposed below the cutting assembly support (360).

3. The medical device assembly (1) of any of claims 1 to 2, wherein the cutting blade (380) is a hemispherical cutting blade (380B).

4. The medical device assembly (1) of claim 3, wherein the hemispherical cutting blade (380B) further comprises serrated teeth (383) extending from a circumference of the hemispherical cutting blade (380B).

5. The medical device assembly (1) of claim 4, wherein the serrated teeth (383) have an outer surface angle (θ) defined by an angle between a normal line (N) to the circumference of the hemispherical cutting blade (380B) and an outer surface (S) of a serrated tooth (383A) of the serrated teeth (383).

6. The medical device assembly (1) of claim 5, wherein the outer surface angle (θ) is less than 10 degrees.

7. The medical device assembly (1) of any of claims 3 to 6, wherein the hemispherical cutting blade (380B) further defines an evacuation hole (385) extending through a blade body of the hemispherical cutting blade (380B).

8. The medical device assembly (1) of any of claims 1 to 7, wherein the positioning member (100) further comprises an articulating clamp (119) at the leading end (111), the articulating clamp comprising a first arm (117a) and a second arm (117b), and wherein pins (113a, 113b) extend through aligned pin holes in the orientation bearing (115) and the first arm (117a) and the second arm (117b) to indirectly engage the orientation bearing (115) to the positioning member (100).

9. The medical device assembly (1) of any of claims 1 to 8, further comprising a cannula (500), wherein the drive member (200) is disposed within the cannula (500).

## Patentansprüche

1. Medizinvorrichtungsbaugruppe (1), umfassend:
ein Ausrichtungslager (115), das eine Innenoberfläche (143), die ein Loch (145) definiert, aufweist;
ein Positionierungselement (100), das mit dem Ausrichtungslager (115) in Eingriff steht, wobei das Positionierungselement (100) einen Längskörper (110), der sich zwischen einem vorderen Ende (111) und einem hinteren Ende (112) erstreckt, aufweist;
eine Schneidbaugruppe (350), wobei die Schneidbaugruppe (350) eine Schneidklinge (380) und einen Schneidbaugruppenträger (360) umfasst, und wobei der Schneidbaugruppenträger (360) einen Passabschnitt (363), der konfiguriert ist, um in dem Loch (145) des Ausrichtungslagers (115) drehbar angeordnet zu werden, aufweist;
ein Antriebselement (200), das einen Antriebselementkörper (210), der sich zwischen einem vorderen Antriebselementende (211) und einem hinteren Antriebselementende (212) erstreckt, aufweist, wobei das vordere Antriebselementende (211) konfiguriert ist, um die Schneidbaugruppe (350) in Eingriff zu nehmen, um die Schneidbaugruppe (350) drehbar anzutreiben; und
einen Antriebssitz (361), der in einer zusammengebauten Konfiguration zwischen dem Schneidbaugruppenträger (360) und dem Antriebselement (200) angeordnet ist, wobei der Antriebssitz (361) konfiguriert ist, um mit der Schneidklinge (380) in Austausch zu stehen, wobei sich die Schneidklinge (380) von dem Schneidbaugruppenträger (360) distal erstreckt, wobei das Antriebselement (200) konfiguriert ist, um den Antriebssitz (361) in Eingriff zu nehmen, und wobei der Antriebssitz (361) ein proximales Ende, das eine Keilschnittstelle aufweist, umfasst, die konfiguriert ist, um mit einer komplementären Keilschnittstelle des vorderen Antriebselementendes (211) zu passen;
**dadurch gekennzeichnet, dass:**
die Schneidklinge (380) eine gekrümmte Schneidklinge (380A) ist, wobei sich eine Keilwelle (347) von der Keilschnittstelle des Antriebssitzes (361) distal erstreckt, und wobei distale Keilzähne (348) auf einem distalen Ende der Keilwelle (347) komplementäre Verzahnung (349) der gekrümmten Schneidklinge (380A), die in dem Schneidbaugruppenträger (360) angeordnet ist, in Eingriff nehmen, derart, dass eine Drehbewegung der Keilwelle (347) in eine bogenförmige Bewegung der gekrümmten Schneidklinge (380A) translatiert wird.

2. Medizinvorrichtungsbaugruppe (1) nach Anspruch 1, ferner umfassend ein Femurkopflager (330), das unterhalb des Schneidbaugruppenträgers (360) angeordnet ist.

3. Medizinvorrichtungsbaugruppe (1) nach einem der Ansprüche 1 bis 2, wobei die Schneidklinge (380) eine halbkugelförmige Schneidklinge (380B) ist.

4. Medizinvorrichtungsbaugruppe (1) nach Anspruch 3, wobei die halbkugelförmige Schneidklinge (380B) ferner gezackte Zähne (383), die sich von einem Umfang der halbkugelförmigen Schneidklinge (380B) erstrecken, umfasst.

5. Medizinvorrichtungsbaugruppe (1) nach Anspruch 4, wobei die gezackten Zähne (383) einen Außenoberflächenwinkel (θ), der durch einen Winkel zwischen einer Normale (N) zu dem Umfang der halbkugelförmigen Schneidklinge (380B) und einer Außenoberfläche (S) eines gezackten Zahns (383A) der gezackten Zähne (383) definiert ist, aufweisen.

6. Medizinvorrichtungsbaugruppe (1) nach Anspruch 5, wobei der Außenoberflächenwinkel (θ) weniger als 10 Grad beträgt.

7. Medizinvorrichtungsbaugruppe (1) nach einem der Ansprüche 3 bis 6, wobei die halbkugelförmige Schneidklinge (380B) ferner ein Entleerungsloch (385), das sich durch einen Klingenkörper der halbkugelförmigen Schneidklinge (380B) erstreckt, definiert.

8. Medizinvorrichtungsbaugruppe (1) nach einem der Ansprüche 1 bis 7, wobei das Positionierungselement (100) ferner eine Gelenkklemme (119) an dem vorderen Ende (111) umfasst, die Gelenkklemme umfassend einen ersten Arm (117a) und einen zweiten Arm (117b), und wobei sich Stifte (113a, 113b) durch ausgerichtete Stiftlöcher in dem Ausrichtungslager (115) und dem ersten Arm (117a) und dem zweiten Arm (117b) erstrecken, um das Ausrichtungslager (115) mit dem Positionierungselement (100) indirekt in Eingriff zu nehmen.

9. Medizinvorrichtungsbaugruppe (1) nach einem der Ansprüche 1 bis 8, ferner umfassend eine Kanüle (500), wobei das Antriebselement (200) innerhalb der Kanüle (500) angeordnet ist.

## Revendications

1. Ensemble de dispositif médical (1) comprenant :
un palier d'orientation (115) ayant une surface intérieure (143) définissant un trou (145) ;
un élément de positionnement (100) engagé dans le palier d'orientation (115), l'élément de positionnement (100) ayant un corps longitudinal (110) s'étendant entre une extrémité avant (111) et une extrémité arrière (112) ;
un ensemble de coupe (350), dans lequel l'ensemble de coupe (350) comprend une lame de coupe (380) et un support d'ensemble de coupe (360), et dans lequel le support d'ensemble de coupe (360) a une partie d'accouplement (363) configurée pour être disposée de manière rotative dans le trou (145) du palier d'orientation (115) ;
un élément d'entraînement (200) ayant un corps d'élément d'entraînement (210) s'étendant entre une extrémité avant d'élément d'entraînement (211) et une extrémité arrière d'élément d'entraînement (212), dans lequel l'extrémité avant d'élément d'entraînement (211) est configurée pour engager l'ensemble de coupe (350) afin d'entraîner de manière rotative l'ensemble de coupe (350) ; et
un siège d'entraînement (361) disposé entre le support d'ensemble de coupe (360) et l'élément d'entraînement (200) dans une configuration assemblée, dans lequel le siège d'entraînement (361) est configuré pour communiquer avec la lame de coupe (380), la lame de coupe (380) s'étendant distalement à partir du support d'ensemble de coupe (360), dans lequel l'élément d'entraînement (200) est configuré pour s'engager dans le siège d'entraînement (361), et dans lequel le siège d'entraînement (361) comprend une extrémité proximale ayant une interface clavetée configurée pour s'accoupler avec une interface clavetée complémentaire de l'extrémité avant de l'élément d'entraînement (211) ;
**caractérisé en ce que**
la lame de coupe (380) est une lame de coupe incurvée (380A), dans lequel un arbre cannelé (347) s'étend distalement à partir de l'interface clavetée du siège d'entraînement (361), et dans lequel des dents cannelées distales (348) sur une extrémité distale de l'arbre cannelé (347) engagent des dents dentées complémentaires (349) de la lame de coupe incurvée (380A) disposée dans le support de l'ensemble de coupe (360) de sorte qu'un mouvement de rotation de l'arbre cannelé (347) se traduit par un mouvement en arc de cercle de la lame de coupe incurvée (380A).

2. Ensemble de dispositif médical (1) selon la revendication 1 comprenant en outre un support de tête fémorale (330) disposé sous le support de l'ensemble de coupe (360).

3. Ensemble de dispositif médical (1) selon l'une quelconque des revendications 1 à 2, dans lequel la lame de coupe (380) est une lame de coupe hémisphérique (380B).

4. Ensemble de dispositif médical (1) selon la revendication 3, dans lequel la lame de coupe hémisphérique (380B) comprend en outre des dents dentelées (383) s'étendant sur la circonférence de la lame de coupe hémisphérique (380B).

5. Ensemble de dispositif médical (1) selon la revendication 4, dans lequel les dents dentelées (383) ont un angle de surface extérieure (θ) défini par un angle entre une ligne normale (N) à la circonférence de la lame de coupe hémisphérique (380B) et une surface extérieure (S) d'une dent dentelée (383A) des dents dentelées (383).

6. Ensemble de dispositif médical (1) selon la revendication 5, dans lequel l'angle de la surface extérieure (θ) est inférieur à 10 degrés.

7. Ensemble de dispositif médical (1) selon l'une quelconque des revendications 3 à 6, dans lequel la lame de coupe hémisphérique (380B) définit en outre un trou d'évacuation (385) s'étendant à travers un corps de lame de la lame de coupe hémisphérique (380B).

8. Ensemble de dispositif médical (1) selon l'une quelconque des revendications 1 à 7, dans lequel l'élément de positionnement (100) comprend en outre une pince articulée (119) à l'extrémité avant (111), la pince articulée comprenant un premier bras (117a) et un second bras (117b), et dans lequel des broches (113a, 113b) s'étendent à travers des trous de broche alignés dans le palier d'orientation (115) et le premier bras (117a) et le second bras (117b) afin d'engager indirectement le palier d'orientation (115) à l'élément de positionnement (100).

9. Ensemble de dispositif médical (1) selon l'une quelconque des revendications 1 à 8, comprenant en outre une canule (500), dans lequel l'élément d'entraînement (200) est disposé à l'intérieur de la canule (500).
